(19) Europäisches Patentamt
European Patent Office
Office européen des brevets

(11) EP 0 978 289 A1

# (12) EUROPEAN PATENT APPLICATION

(43) Date of publication:
**09.02.2000 Bulletin 2000/06**

(21) Application number: **99107457.6**

(22) Date of filing: **28.04.1999**

(51) Int. Cl.$^7$: **A61K 47/32**, A61K 33/18, A61P 31/12, A61P 31/14, A61P 31/18, A61P 31/20 // (A61K47/32, 33:18, 33:14, 31:715, 31:702)

(84) Designated Contracting States:
**AT BE CH CY DE DK ES FI FR GB GR IE IT LI LU MC NL PT SE**
Designated Extension States:
**AL LT LV MK RO SI**

(30) Priority: **29.07.1998 CZ 0238898**

(71) Applicants:
- **Iljin, Aleksandr Ivanovic**
  **Almaty (KZ)**
- **Sarkesjan, Albert Artusevic**
  **Almaty (KZ)**
- **Borisov, Pavel Georgievic**
  **Almaty (KZ)**
- **Bozkov, Jevgenij Alexejevic**
  **Oktjabr Almaatinskaja oblast (KZ)**
- **Arakeljan, Norjan Garibaldevic**
  **Almaty (KZ)**
- **Verbkin, Anatolij Vasiljevic**
  **Almaty (KZ)**
- **Franc, Petr**
  **Praha 9 (CZ)**

(72) Inventors:
- **Iljin, Aleksandr Ivanovic**
  **Almaty (KZ)**
- **Sarkesjan, Albert Artusevic**
  **Almaty (KZ)**
- **Borisov, Pavel Georgievic**
  **Almaty (KZ)**
- **Bozkov, Jevgenij Alexejevic**
  **Oktjabr Almaatinskaja oblast (KZ)**
- **Arakeljan, Norjan Garibaldevic**
  **Almaty (KZ)**
- **Verbkin, Anatolij Vasiljevic**
  **Almaty (KZ)**
- **Franc, Petr**
  **Praha 9 (CZ)**

(74) Representative:
**Kruspig, Volkmar, Dipl.-Ing. et al**
**Patentanwälte**
**Meissner, Bolte & Partner**
**Postfach 86 06 24**
**81633 München (DE)**

## (54) Virucide drug containing iodine

(57) The invention relates to the field of medicine, particularly to drug of a wide range of virucide effect specifically inhibiting and /or virucide effect on DNA- and RNA containing viruses.

It may be used for treating for diseases of viral nature of mammals, including humans.

The claimed drug contains, as an active agent, iodine, potassium or sodium iodide, lithium chloride, a synthetic water-soluble gel-forming polymer, as well as natural polymers, mono- and oligosaccharides.

The virucide drug also exhibits an immunomodulating and immunostimulating effect.

EP 0 978 289 A1

## Description

The invention relates to the field of medicine, particularly to drugs of a wide range of virucidal effect affecting DNA- and RNA-containing viruses in mammals, including humans. It may be used to control infectious diseases of viral nature.

Many drugs have been offered during the last three decades for treatment for viral diseases. However, in the past, therapeutic drug tests led to erroneous conclusions because of the lack of correct assessment of the natural development of most viral infections, the lack of knowledge of the mechanism of affection of the living cells of the organism by a virus. Recently, more detailed studies and the development of specific viral diagnostic techniques, a wider biochemical knowledge of virus replication processes, as well as a better understanding of the virus behavior in specific host cell populations have resulted in a tendency to develop more efficacious drugs to be used against viral infections. The discovery of endogenic interferon and its antiviral activity was an outstanding event. It has now been found that the effect of a number of immunity stimulating and antiviral drugs is connected with their interferonogenic activity, i.e. the ability of stimulating the formation of endogenic interferon (Mashkovski M.D. Lekarstvenniye sredstva: Posobiye dla vrachey. - T.2. - Kharkov: "Torsing", 1998. -P. 349).

Natural (leukocytic human) interferon is still being used for the prevention and treatment for influenza and other viral infections. Among the most efficacious antiviral drugs one should single out remantadin which is used for treatment for diseases caused by the virus of influenza, adenoviruses, picornaviruses and other (Kukain R.A. et al. Antivirusnaya aktivnost i mekhanism deistviya razlichnykh khimicheskikh soyedineniy. - Riga: "Znaniye", 1979).

Mutability is a dangerous feature of viruses. Viral epidemics such as Spanish flu, Hong Kong flu and, finally, AIDS which is a retrovirus, were caused by animal viruses, which nevertheless affect humans.

There is a liposomic pharmaceutical composition which contains lipid and polyene, for instance, nystatin and amphotericin B are used for the treatment for AIDS.

However, liposomic compositions are selectively toxic for HIV-1- infected cells (WO 92/118415, class A61K9/127, 31/7, 1992). Rapamicin or its analogue is also used in the treatment for AIDS, which arrests the HIV infection development and slows down its spread in mammals (WO 94/05300, class A61K31/71, 1994).

There is also a pharmaceutical drug which contains benanomicin A or B as an active agent for inhibiting the infection with the AIDS virus (EP 0356330A, class 4A61K31/71, published on 15.09.1990). The disadvantage of the above drugs used for curing or /and inhibiting AIDS is that they cause a number of side effects. 3'-azido-2'3'-didezoxotimidin (also referred to as azydotimidin or zydovudin). This drug has a positive effect against the pathogenic retroviral infection, but, unfortunately, one cannot say that the AIDS virus can be totally destroyed with the help of this drug (V mire nauki: Illustrative magazine. - Moscow.: Mir, 1988, pp. 80-81). The closest in technical essence and result achieved is the drug which has a bactericide and virucide effect and contains iodine, potassium or sodium iodide, a synthetic water-soluble polymer, natural polymers such as polysaccharides and mono-and oligosaccharides in the following proportions, g/l:

| | |
|---|---|
| Iodine | 6-10 |
| Potassium or sodium iodide | 9-15 |
| Synthetic water-soluble polymer | 2-4 |
| Natural polymer (polysaccharides and mono- and oligosaccharides | 8-120 |
| Water | the rest |

( 5435, A61K33/18, A61K31/715)

The disadvantage of this drug is its relatively high toxicity.

It is difficult to affect the viruses that have penetrated into the cell, as the cell itself reliably protects them against any effect. It is in the cell that the adapted virus performs its destructive action. The problem is to suppress specific processes of the biological synthesis of virus particles on the molecular level, possibly without any harm to the vital activity of the cells uninfected by the virus.

Thus, the task is to find a drug which would be capable of destroying the virus both outside and inside the infected cell without destroying the cell itself.

In addition, this drug should have a wide range of effect and low toxicity. The task set has been solved with the help of the drug developed by the authors of this invention.

The authors propose a virucide drug acting, in particular, on the DNA- and RNA-containing viruses of mammals, including humans, which contains iodine, potassium or sodium iodide, a synthetic water-soluble polymer, natural polymers such as polysaccharides, and mono- and oligosaccharides and water, and, in addition, lithium chloride in the following proportions, g/l:

| | |
|---|---|
| Iodine | 0.8-25 |
| Potassium or sodium iodide | 1.2-38 |
| Lithium chloride | 0.1-20 |
| Synthetic water-soluble polymer | 0 ,0 1-6 |
| Natural polymers (polysaccharides) and Mono- and oligosaccharides | 8-400 |
| Water | the rest. |

The choice of concentration ranges was not accidental. All parameters of the proposed virucide drug are necessary and sufficient for achieving the set goal. None of these can be omitted or replaced without failure to achieve the technical result. The choice of both qualitative and quantitative composition of the ingredients of the proposed drug was based on numerous experimental data. The qualitative ingredient composition is obvious from the viewpoint of their being well-known in the literature; the authors of the claimed drug, however, came to the conclusion that together with the other components each component of the drug exhibits properties which are not so obvious. Since iodine is the chemically and biologically active element, at its concentration of less than 0.8 g/l iodine hydrolyses as follows:

$$I_2 + H_2 \rightarrow HI + HOI$$

$$\downarrow$$

$$HI + O$$

which means the drug is not to be stored.

At iodine concentrations greater than 25 g/l the structural formation of complex iodine associates with the drug components produces jelly-like systems which are not covenant for medical use. In addition, from time to time there occur halogenation reactions of mono-, oligo - and polysaccharides, which increases the drug toxicity. The concentration ranges for the other components are used to make sure that the reactions of complex formation, association and aggregation occur in the process of drug preparation in order to ensure its virucide properties.

Synthetic water-soluble polymers (e.g., polyvinyl alcohol, poly-N-vinylpyrrolidone) when injected in the blood perform the function of plasma-substitutes, sorbents (e.g. toxins) or prolongators.

In our case, the synthetic water-soluble polymer performs the jelly-forming function.

It should be noted that one of the important aspects in the choice of components for the drug is that the complex compound of iodine and low- and high-molecular ligands, which in their turn, formassociates, play the role of a matrix. Their aqueous solution has some properties of a gel.

It is known that carbohydrates, including mono- and polysaccharides, and proteins and lipids are constituents of the biological membranes forming interfaces between or inside cells.

Proteins found in the membranes are usually enzymes (Coleman R. Biochem. Biophys. Acta. - 1973.- 300.- 1). In turn, the membranes ensure the necessary spatial interactions between enzymes, as a result, the product of the reaction catalyzed by one enzyme become a substrate for the adjacent other enzyme.

Hormones play an important role in regulating metabolic processes at the intracellular level. The binding of hormones with their receptors is similar to the interaction of an enzyme and its substrate.

The formation of enzyme-substrate and hormone-receptor complexes assumes that molecules "recognize" each other. At the higher level of organization this capability is exhibited by cells. Thus, leukocytes in the blood flow recognize and destroy alien cells, for instance bacterial cells, but do not attack the blood's own cells. Recognition is also exhibited in contact inhibition: some cells of higher organisms continue to split in a nutrient medium until they contact other cells. Cancer cells continue to split under the same conditions.

Processes of cell recognition depend on the mobility of membrane components (Bretscher MS., Raff MC. - Nature. - 1975.- 258.- 43).

Carbohydrates, including mono-, oligo- and polysaccharides chosen by the authors of this invention as natural

polymers, constitute an extremely important class of natural compounds. The importance of carbohydrates in biology and medicine is in the dominant role which they play in the majority of living organisms and the complexity of their functions. Carbohydrates participate in the majority of biochemical processes in the form of high-molecular particles, though many biological liquids contain mono- and oligosaccharides (Comprehensive Organic Chemistry/ Edited by E. Haslam. V.5. Biological Compounds. Pergamon Press, 19).

Mono-, oligo- and polysaccharides contained in the drug are not alien to the organism of mammals, including humans, and play an important role in the process of cell recognition.

Iodine contained in the drug both in the molecular form and as salts (potassium or sodium iodide) exist in the drug in several active forms due to the reactions of complex formation:

$$I_2 + KI \leftrightarrow KI_3 \leftrightarrow K^+ + I_3^- \qquad (1)$$

$$L + x\,I_2 \leftrightarrow L \bullet xI_2 \leftrightarrow [L \bullet xI]^+ + (x-1)\,I_3^- \qquad (2)$$

$$L + I_3^- \leftrightarrow [L \bullet xI_3]^- \qquad (3)$$

where L is a ligand (mono-, oligo-, polysaccharide and a synthetic water-soluble polymer).

The association of the complex compounds produced in reactions (1) leads to the gel formation in the drug. The gel forming function of the synthetic water-soluble polymer is of great importance.

At present the liquid mosaic model of biological membranes has become commonly accepted, which assumes that under physiological conditions a membrane is fluid, rather than static. (Singer S.J. Ann. New York Acad. Sci.- 1972.- 195.- 16).

At the characteristic temperature, bilayers that form the membrane structure and are made up of phospholipids, as well as glyco- and sulfolipids, undergo a pronounced phase transition from a relatively rigid jelly-like state to a fluid liquid crystal state. As a result, the membrane components are capable of diffusing into the membrane plane. The diffusion coefficient for protein with a molecular weight of $3 \bullet 10^{10}$ included into the membrane is $3 \bullet 10^{10}$ $cm^2/s$ (Edidian M. Ann. Rev. Biophys. Bioeng.- 1974.-3.-179). Lateral diffusion in biological membranes is to a large extent a regulated process.

Transverse movement of membrane components between the inner and the outer halves of the bilayer occurs in time, but the presence of other biological membrane components may sharply increase the velocity of this movement (up to $t_{1/2}$ 5 min) (Marsh D. Essays Biochem.- 1975.-11.- 139).

Iodine, its various forms contained in the drug, is capable of interacting with practically all classes of substances, contained in the organism of mammals, including humans, as well as in the membrane and the cell itself: proteins, carbohydrates, amino acids, glyco- and phospholipids, hormones, enzymes, vitamins etc. With this, there occur reactions of complex formation and haligenation; however, due to the sequence of biochemical processes in a living organism, the interaction of one of the active iodine forms results in the production of a new active form of iodine which is a constituent of other complexes, e.g. iodine-enzyme, iodine-hormone, iodine-biological membrane, iodine-protein etc.

Each newly formed complex is biologically active and is "friendly" to the living organism of mammals, including humans.

Due to the mechanism of recognition and specific chemical interaction, iodine contained in the drug, as well as iodine complexes forming in the organism, halogenate (iodize) DNA- and RNA-containing viruses both inside and outside the cell.

Halogenation occurs primarily through the substitution of the hydrogen atom in the peptide bond of the virus molecule:

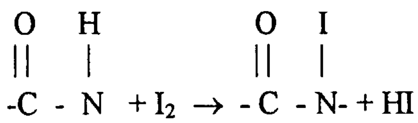

(4)

and the substitution of the SH group in the virus molecular chain

$$R\text{-}SH + I_2 + H_2O \rightarrow R\text{-}I + H_2S + HOI \quad (5)$$
$$HOI \downarrow HI + O$$

Halogenation produces new substances which do not have the properties of the initial virus. This is the basis of the specific virucide effect of the drug.
The sequence of the biological reactions which occur upon introduction of the drug into the organism of a mammal, including a human being, is explained by the immunomodulating, immunostimulating action of the drug.
Lithium chloride introduced into the drug composition plays a role which is unusual for halogenides of alkali metals. It improves the conductivity in cellular membranes, facilitates a better lateral diffusion in the bilayer of cellular membranes due to the small size of the lithium cation and its strong coordination of the donor atoms of mono-, oligo- and polysaccharides.
The proposed virucide drug affecting, in particular, DNA- and RNA-containing viruses is a dark blue water solution of molecular and ionic complexes of iodine and natural mono-, oligo- and polysaccharides. The melting temperature of the drug is - 1.51°C, density is 1.048 g/$cm^3$, weight percent of dry matter is at least 25%. The virucide drug has pH 6-7. All its components are dissolved in the izotonic solution of sodium chloride.
The virucide drug is low toxic. $LD_{100}$ is 75g/kg of live weight (for white mice) in peroral administration.
The virucide drug has a wide range of action. It is effective against infections caused by the viruses of poliomyelitis, foot and mouth disease, influenza, measles (RNA-containing viruses); those caused by the viruses of herpes, sheep pox, ecthyma etc. (DNA-containing viruses).
The drug is not toxic for cellular cultures if diluted with water in 1:20 and 1:100 proportions.
The antiviral effect of the drug is exhibited at all levels in cases of single, preliminary and subsequent application with respect to viruses.
The drug is an effective means for the chemotherapy of viral infections.
The drug is capable of stimulating the formation of antibodies in lymphocyte cultures.
The virucide drug is designed for intravenous, intramuscular, intra-abdominal and hypodermical applications to mammals, including humans. It may also be
administered perorally etc. The technique of the drug production is based on the reactions of complex formation between iodine and poly-functional low- and high-molecular ligands.
The technique itself of producing the antiviral drug is as follows. 9 g of potassium iodide is dissolved in 100 ml of water, then 6 g of crystalline iodine is added and the solution is stirred until complete dissolution of iodine. 2 g of polyvinyl alcohol is dissolved in 100 ml of water separately. 30 g of a mono-, oligo-and polysaccharide mixture is dissolved in 200 ml of water separately. The obtained solutions are mixed in the following sequence: the solution of the mono-, oligo- and polysaccharide mixture is added to the polyvinyl alcohol solution, and then the iodine and potassium iodide solution is added. Then up to 1 litre of distilled water is added, with prior addition of 9 g of sodium chloride and 2 g of lithium chloride.
The above technique with the same sequence is used to prepare the drug solutions taking into account the concentrations of the components claimed.
The antiviral activity of the claimed drug was studied with respect to the influenza A virus, strain A/Aichi 2/68 ($H_3N_2$) - a representative of orthomicroviruses, the virus of human herpes simplex of second serotype (the family of herpes viruses), the virus of encephalomyocarditis (the family of picornaviruses), vaccine attenuated poliovirus of type III, Sabin strain, under the production conditions in the case of foot and mouth disease of sheep.
The toxicity of the virucide drug was studied on the cellular cultures of mice and chicken embryos, as well as with respect to lymphocytes.
The intertwined line of the adenocarcinoma of human laryHep-2 and the initial culture of KE and FEK fibroplasts. Cells were cultivated in the Igla medium with glutamin and ox serum (Hep-2) and in medium 199 with serum (FEK).
The influence of the proposed drug on the induction of the humoral and cellular immune response of human lymphocytes in the culture was studied with the help of the method described in the journal Neirokhimiya.- 1998.-V.15, issue 1.-pp.45-50).
The following standard methods were used in studies of antiviral activity:

- Virus titration on cellular cultures by cytopathic effect;
- Titre determination by the Kerber method;

- Virus titration on mice by the Reid and Mench method;
- Virus titration on chicken embryos;
- In the hemagglutination reaction.

The virucide drug was used in the form of 1:5 and 1:100 aqueous solutions prepared ex tempore.
Official antiviral drugs -remantadin and acyclovir- were used as positive reference on mice and chicken embryos.
Results of physico-chemical experiments and biological tests were statistically processed using the methods described in GPh XI USSR, pp. 199-251. In order to confirm the claimed component proportions, the compositions used in tests are as follows, g/l:

| | | |
|---|---|---|
| I. | Crystalline iodine | 0.8 |
| | Potassium (sodium) iodide | 1.2 |
| | Lithium chloride | 0.1 |
| | Natural mono-, oligo- and polysaccharides | 100 |
| | Water | the rest |
| II. | Crystalline iodine | 8 |
| | Potassium (sodium) iodide | 12 |
| | Lithium chloride | 2 |
| | Water-soluble synthetic polymer | 3 |
| | Natural mono-, oligo- and polysaccharides | 100 |
| | Water | the rest |
| III. | Crystalline iodine | 25 |
| | Potassium (sodium) iodide | 38 |
| | Lithium chloride | 20 |
| | Water-soluble synthetic polymer | 6 |
| | Natural mono-, oligo- and polysaccharides | 400 |
| | Water | the rest |

The natural polysaccharides used are all natural polysaccharides, including antibiotics of this class of substances and their derivatives which react with iodine to produce water-soluble complex compounds within the claimed concentration range of iodine and other components. The used synthetic water-soluble polymers which have gel-forming properties include polymers permitted for use by the International Parmacopeia and the USSR Parmacopeia, Issue XI, which form water-soluble complexes with iodine within the claimed concentration ranges of iodine and other components.
The preventive and therapeutic effects of the claimed drug were studied on white mice of 12-14 g of weight, in which experimental influenza infection was reproduced by way of intranasal introduction of a virus suspension containing 100 $LD_{50}$ in the volume of 0.05 ml after the virus titration and determination of $LD_{50.}$ The influenza virus titre in mice tables 1-3.
Remantadin, one of the most effective drugs against the influenza A virus, was applied to mice as a positive reference.
As the data in tables 1-3 show, the claimed drug in 1:10 and 1:20 dilutions provide a statistically reliable protection to animals against influenza infection when applied 2 hours before getting infected.
The low initial concentration of iodine in the drug, according to example 1 (table 2), of course, does not make it possible to obtain a positive effect with further dilutions of the drug.
Table 4 gives data of the studies of the therapeutic effect of the drug on influenza infection in mice. Dose of the drug, which exhibited an antiviral effect when applied for preventive purposes, were applied to mice intra-abdominally 2 hours after they got infected with the influenza virus.

Examination of the data in table 4 indicated a reliable decrease of lethality upon administration of the initial drug and the drug in 1:10 dilution.

Table 5 shows data of the studies of the capability of the claim drug in 1:20 and 1:40 dilutions to neutralize 100 $TCD_{50}$ of the encephalocarditis virus when administed 1 hour before and after getting infected. The drug capability to neutralize the virus was judged by the degree of the cytopatic effect in test and reference tubes containing a monolayer of Hep-2 cells the cultural liquid of which was titrated.

As the data in table 5 indicate, even when the drug was applied in 1:40 dilution after infection with the virus at a dose of 100 $TCD_{50}$, the difference between test and reference was 3.5-4.0 log, which is an evidence of a significant suppression of the virus.

The antiviral effect of the claimed drug on the reproduction of the virus herpes simplex (VHS) was studied on developing chicken embryos, on whose chorioallantoic membrane the VHS reproduction is exhibited as proliferation patches.

The test results given in table 6 indicate that the virus reproduction is inhibited by the drug in 1:10 dilution. The difference between the virus titre in the test and the reference exceeds 2 log, which evidences that the virus titres in tests 1 and 2 are suppressed by 60% and 65%, respectively.

Data on the effect of the claimed drug on the induction of humoral immune response of human tonsillar lymphocytes to Iersinia enterocolitica and Staphylococcus aureus are given in table 7.

The data in table 7 show that the claimed drug is capable of stimulating the formation of antibodies to Iersinia enterocolitica and Staphylococcus aureus in the lymphocyte culture at concentrations ranging from 1:10 to 1:100

The claimed drug also stimulates the bactericide activity of human lymphocytes to Iersinia enterocolitica and Staphylococcus aureus.

The effect of the claimed drug against the foot and mouth disease of sheep was studied under production conditions. Obtained results are shown in tables 8 and 9.

The data in tables 8 and 9 confirm the above data on the preventive effect of the drug, since no animals with the foot and mouth clinical picture were identified after two injections of the drug.

The data in tables 8 and 9 also provide evidence of a reliable therapeutic effect of the virucide drug since the loss of animals was terminated after two injection of the drug, while the remaining sick animals recuperated in 7-10 days. The total loss of animals in the test group was 1.83%, while that in the reference group was 14% in spite of quarantine measures.

Table 10 gives data on the therapeutic effect of the claimed drug in human diseases caused by both DNA- and RNA-containing viruses. The drug was applied to volunteers.

The Data in table 10 show that the drug is efficacious in all cases, especially against infections of short duration (influenza, diarrhea, ARD, foot and mouth). No complications were observed in any case.

The above examples are without limitation but an illustration of the essence of the invention.

Thus, test data obtained in vitro and in vivo on the antiviral effect of the claimed drug, the absence of toxicity, capability of destroying the virus outside and inside the cell, its both preventive and therapeutic effect, a wide range of action allow the drug to be characterized as an efficacious means of drug therapy for virus infections.

Table 1

| **The effect of preventive application of the virucide drug on the outcome of experimental influenza infection in mice** | | | | | | |
|---|---|---|---|---|---|---|
| **Drug composition as in example 1**. | | | | | | |
| **Test conditions** | **Drug dosage, mg/kg** | **Number of iodine molecules per 1 l.** | **Ratio of 1 IV IU to number of iodine molecules** | **Number of mice** | **Absolute number of dead** | **% lethality** |
| $D^1(1/10) + IV^2$ | 12,32 | $5 \cdot 10^{19}$ | $1:5 \cdot 10^{10}$ | 100 | 12/100 | 12 |
| D(1/20) + IV | 6.16 | $2.5 \cdot 10^{19}$ | $1:2.5 \cdot 10^{10}$ | 100 | 28/100 | 28 |
| D(1/40) + IV | 3.08 | $1.25 \cdot 10^{19}$ | $1:1.25 \cdot 10^{10}$ | 100 | 50/100 | 50 |
| Remantadin + IV | 1.66 | $7.9 \cdot 10^{18}$ | $1: 7.9 \cdot 10^{9}$ | 100 | 16/100 | 16 |

Table 1 (continued)

| The effect of preventive application of the virucide drug on the outcome of experimental influenza infection in mice | | | | | | |
|---|---|---|---|---|---|---|
| **Drug composition as in example 1**. | | | | | | |
| IV | | | | 100 | 70/100 | 70 |
| **Notes**:<br>1. D is the drug (virucide drug);<br>2. IV is the influenza virus;<br>3. In terms of iodine;<br>4. IU is the infection unit | | | | | | |

Table 2

| The effect of preventive application of the virucide drug on the outcome of experimental influenza infection in mice | | | | | | |
|---|---|---|---|---|---|---|
| **Drug composition as in example 1**. | | | | | | |
| **Test conditions** | **Drug dosage, mg/kg** | **Number of iodine molecules per 1l.** | **Ratio of 1 IV IU to number of iodine molecules** | **Number of mice** | **Absolute number of dead** | **% lethality** |
| D[1] + IV[2] | 3.69 | $1.5 \cdot 10^{19}$ | $1:1.5 \cdot 10^{10}$ | 100 | 38/100 | 38 |
| D(1/10) + IV | 0.369 | $1.5 \cdot 10^{18}$ | $1: 1.5 \cdot 10^{9}$ | 100 | 60/100 | 60 |
| Remantadin | 1.66 | $7.90 \cdot 10^{18}$ | $1: 7.90 \cdot 10^{9}$ | 100 | 16/100 | 16 |
| IV | | | | 100 | 70/100 | 70 |
| **Notes**:<br>1. D is the drug (virucide drug);<br>2. IV is the influenza virus;<br>3. In terms of iodine;<br>4. IU is the infection unit. | | | | | | |

Table 3

| The effect of preventive application of the virucide drug on the outcome of experimental influenza infection in mice | | | | | | |
|---|---|---|---|---|---|---|
| **Drug composition as in example 3**. | | | | | | |
| **Test conditions** | **Drug dosage, mg/kg** | **Number of iodine molecules per 1l.** | **Ratio of 1 IV IU to number of iodine molecules** | **Number of mice** | **Absolute number of dead** | **% lethality** |
| D[1](1/31) + IV[2] | 12,32 | $5 \cdot 10^{19}$ | $1: 5 \cdot 10^{10}$ | 100 | 11/100 | 11 |
| D(1/62) + IV | 6.16 | $2.5 \cdot 10^{19}$ | $1: 2.5 \cdot 10^{10}$ | 100 | 27/100 | 27 |
| D(1/124) + IV | 3.08 | $1.25 \cdot 10^{19}$ | $1: 1.25 \cdot 10^{10}$ | 100 | 51/100 | 51 |
| Remantadin + IV | 1.66 | $7.9 \cdot 10^{18}$ | $1:7.9 \cdot 10^{9}$ | 100 | 16/100 | 16 |

Table 3 (continued)

| **The effect of preventive application of the virucide drug on the outcome of experimental influenza infection in mice** | | | | | | |
|---|---|---|---|---|---|---|
| **Drug composition as in example 3**. | | | | | | |
| IV | | | | 100 | 70/100 | 70 |
| **Notes**:<br>1. D is the drug (virucide drug);<br>2. IV is the influenza virus;<br>3. In terms of iodine;<br>4. IU is the infection unit. | | | | | | |

Table 4

| **The therapeutic effect of the virucide drug on influenza infection in mice** | | | | | | |
|---|---|---|---|---|---|---|
| **Drug composition as in example 2**. | | | | | | |
| **Test conditions** | **Drug dosage, mg/kg** | **Number of iodine molecules per 1l.** | **Ratio of 1 IV IU to number of iodine molecules** | **Number of mice** | **Absolute number of dead** | **% lethality** |
| IV[1] + D[2] | 123.2 | $5 \cdot 10^{20}$ | 1: $5 \cdot 10^{11}$ | 100 | 3/100 | 3 |
| IV + D(1/10) | 12.32 | $5 \cdot 10^{19}$ | 1: $5 \cdot 10^{10}$ | 100 | 21/100 | 21 |
| IV + D(1/20) | 6.16 | $2.5 \cdot 10^{19}$ | 1: $2.5 \cdot 10^{10}$ | 100 | 42/100 | 42 |
| IV + Remantadin | 1.66 | $7.9 \cdot 10^{18}$ | 1: $7.9 \cdot 10^{9}$ | 100 | 20/100 | 20 |
| IV | | | | 100 | 60/100 | 60 |
| **Notes**:<br>1. D is the drug (virucide drug);<br>2. IV is the influenza virus;<br>3. In terms of iodine;<br>4. IU is the infection unit. | | | | | | |

Table 5

| **The effect of preliminary and subsequent application of the virucide drug on the reproduction of the encephalomyocarditis virus (EMV) in Hep-2 culture cells**. | | | | | | |
|---|---|---|---|---|---|---|
| **Drug composition as in example 2**. | | | | | | |
| **Test conditions** | **CPD expression** | | **Sample titration results** | | **Degree of inhibition in log** | |
| | **Test 1** | **Test 2** | **Test 1** | **Test 2** | **Test 1** | **Test 2** |
| D(1/20) + EMV | 0/4 | 1/4 | 1.0 | 2.0 | 5.5 | 4.5 |
| D(1/40) + EMV | 0/4 | 1/4 | 0.75 | 2.25 | 5.75 | 4.25 |
| EMV + D (1/20) | 1/4 | 1/4 | 1.5 | 1.75 | 5.0 | 4.75 |
| EMV + D (1/40) | 2/4 | 3/4 | 2.5 | 3.0 | 4.0 | 3.5 |
| Reference EMV | 4/4 | 4/4 | 6.5 | 6.5 | | |

Table 6

| Results of the study of the inhibiting effect of the virucide drug on the reproduction of the herpes simplex virus (HSV) in chicken embryos (CE) | | | | | | | | | |
|---|---|---|---|---|---|---|---|---|---|
| | | Number of infected CE | | | | HSV titres in log | | | |
| Virus dilution | Number of CE | reference | | test | | reference | | test | |
| | | 1 | 2 | 1 | 2 | 1 | 2 | 1 | 2 |
| $10^{-1}$ | 100 | 100/100 | 75/100 | 50/100 | 50/100 | 3.75 | 3.5 | 1.5 | 1.25 |
| $10^{-2}$ | 100 | 100/100 | 75/100 | 50/100 | 25/100 | | | | |
| $10^{-3}$ | 100 | 75/100 | 50/100 | 0/100 | 0/100 | | | | |
| $10^{-4}$ | 100 | 50/100 | 50/100 | 0/100 | 0/100 | | | | |
| $10^{-5}$ | 100 | 0/100 | 25/100 | 0/100 | 0/100 | | | | |

Table 7

| The effect of different drug comcentration on the formation of antibody-forming cells (AFC) in the culture to antigenes. | | |
|---|---|---|
| Drug composition as in example 2. | | |
| Test conditions | Number of antigene-specific AFC in (in terms of 1 ($10^6$ cells) | |
| | Iersinia enterocolitica | Staphylococcus aureus |
| reference | 54 | 39 |
| PWM* | 155 | 74 |
| Drug dilution | | |
| 1:10 | 153 | 102 |
| 1:20 | 129 | 148 |
| 1:50 | 114 | 153 |
| 1:100 | 89 | 75 |

Note: * PWM - poke weed mitogene.

Table 8

| Results of the virucide drug effect against the foot and mouth of sheep. | | | | | | | |
|---|---|---|---|---|---|---|---|
| **Drug composition as in example 2.**<br>**1st application of the drug.** | | | | | | | |
| | | **Number of sick animals** | | **Drug dosage -0.1 ml/kg of live weight Intramuscular** | | | |
| | **Number of sick animals** | **head** | **%** | **number of dead animals** | | **number of sick animals** | |
| | | | | **head** | **%** | **head** | **%** |
| Test | 1200 | 52 | 4.3 | 20 | 1.7 | 5 | 0.42 |
| Reference | 500 | 25 | 5.0 | 15 | 3.0 | 20 | 4.1 |

Table 9

| Results of the virucide drug effect against the foot and mouth of sheep. | | | | | | | |
|---|---|---|---|---|---|---|---|
| **Drug composition as in example 2.**<br>**2nd application of the drug after 24 hours** | | | | | | | |
| | | **Number of sick animals** | | **Drug dosage -0.1 ml/kg of live weight Intramuscular** | | | |
| | **Number of sick animals** | **head** | **%** | **number of dead animals** | | **number of sick animals** | |
| | | | | **head** | **%** | **head** | **%** |
| Test | 1200 | 52 | 4.3 | 2 | 0.17 | - | - |
| Reference | 500 | 25 | 5.0 | 20 | 4.3 | 5 | 5.4* |

* After that the reference group was vaccinated, total loss of animals was 14%.

Table 10

| Results of the therapeutic effect of the virucide drug in case of diseases of viral nature. Application- intramuscular. Drug composition as in example 2. | | | | | |
|---|---|---|---|---|---|
| **Virus family** | **Disease** | **Number of volunteers** | **Daily dose, ml/kg** | **Duration of treatment, days** | **Number of doses** |
| **DNA-containing viruses** | | | | | |
| Herpetoviridae Herpetovirus 2 | Herpes | 22 | 0.2 | 3-5 | 3 |
| Adenoviridae Mastadenovirus | Catarrh of upper respiratory tracts | 15 | 0.2 | 2-3 | 2-3 |
| **RNA-containing viruses** | | | | | |
| Reoviridae Rotavirus | Diarrhea | 18 | 0.2 | 1-3 | 1-3 |
| Coronaviridae Coronavirus | Acute respiratory Disease (ARD) | 50 | 0.2 | 1-3 | 1-3 |
| Ortomyxoviridae Influenzavirus A | Influenza | 35 | 0.2 | 1-3 | 1-2 |
| Picornaviridae Aphtovirus | Foot and mouth | 3 | 0.2 | 2-3 | 2 |

## Claims

**1.** A virucide drug affecting, particularly, DNA- and RNA-containing viruses of mammals, including humans, - which contains iodine, potassium or sodium iodide, a synthetic water-soluble polymer, natural polymers, for instance polysaccharides, and mono- and oligosaccharides and water, containing, in addition, lithium chloride, in the following contents, g/l:

| | |
|---|---|
| Iodine | 0.8-25 |
| Potassium or sodium iodide | 1.2-38 |
| Lithium chloride | 0.1-20 |
| Synthetic water-soluble polymer | 0 , 01-6 |
| Natural polymers (polysaccharides) and mono- and oligosaccharides | 8-400 |
| Water | the rest. |

**2.** A drug according to claim 1 exhibiting an immunomodulating and immunostimulating effect.

European Patent Office

# EUROPEAN SEARCH REPORT

Application Number
EP 99 10 7457

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 0 823 996 A (YUUGEN KAISHA AICS LAB SANGYO) 18 February 1998 (1998-02-18)<br>* claims 1,2,4,12,13 *<br>* column 5, line 18 - column 6, line 21 * | 1,2 | A61K47/32<br>A61K33/18<br>A61P31/12<br>A61P31/14<br>A61P31/18<br>A61P31/20<br>//(A61K47/32, 33:18,33:14, 31:715,31:702) |
| Y | US 5 545 401 A (SHANBROM EDWARD) 13 August 1996 (1996-08-13)<br>* claims 1-4 * | 1,2 | |
| A | WO 95 28165 A (UNIV KENTUCKY RES FOUND) 26 October 1995 (1995-10-26)<br>* claims 1-14 * | 1,2 | |
| | | | TECHNICAL FIELDS SEARCHED (Int.Cl.7) |
| | | | A61K |

The present search report has been drawn up for all claims

| Place of search | Date of completion of the search | Examiner |
|---|---|---|
| MUNICH | 15 December 1999 | Uiber, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 10 7457

This annex lists the patent family members relating to the patent documents cited in the above-mentioned European search report.
The members are as contained in the European Patent Office EDP file on
The European Patent Office is in no way liable for these particulars which are merely given for the purpose of information.

15-12-1999

| Patent document cited in search report | Publication date | Patent family member(s) | Publication date |
|---|---|---|---|
| EP 0823996 A | 18-02-1998 | JP 10109938 A | 28-04-1998 |
| | | AU 2848597 A | 19-02-1998 |
| | | CA 2209540 A | 15-02-1998 |
| | | DE 823996 T | 24-09-1998 |
| | | PL 321476 A | 16-02-1998 |
| | | SK 111497 A | 04-03-1998 |
| US 5545401 A | 13-08-1996 | NONE | |
| WO 9528165 A | 26-10-1995 | US 5492692 A | 20-02-1996 |
| | | AU 1128395 A | 10-11-1995 |
| | | CA 2188319 A | 26-10-1995 |

EPO FORM P0459

For more details about this annex : see Official Journal of the European Patent Office, No. 12/82